# EUROPEAN PATENT SPECIFICATION

(11) **EP 2 051 592 B1**
(45) Date of publication and mention of the grant of the patent: **27.03.2013**
(21) Application number: 07787756.1
(22) Date of filing: 20.07.2007
(51) Int. Cl.: A23K 1/14, A23K 1/16, A23K 1/18

(54) **DRY FOOD TO REDUCE DOGS' APPETITES**
TROCKENFUTTER FÜR DIE APPETITZÜGELUNG BEI HUNDEN
ALIMENT SEC POUR REDUIRE L'APPETIT DES CHIENS

(30) Priority: 21.07.2006 FR 0606681
(43) Date of publication of application: 29.04.2009
(73) Proprietor: Royal Canin S.A., 30470 Aimargues (FR)
(72) Inventor: BIOURGE, Vincent, F-30250 Villevielle (FR); GAYS, Jérôme, F-34070 Montpellier (FR); BISSOT, Thomas, F-34070 Montpellier (FR); ECOCHARD, Claude, F-30670 Aigues-vives (FR); SERGHERAERT, Renaud, F-34690 Fabregues (FR); WEBER, Mickael, F-34400 Lunel (FR); SOULARD, Yannick, F-34160 Beaulieu (FR)
(74) Representative: Cornish, Kristina Victoria Joy
(86) International application number: PCT/EP2007/057501
(87) International publication number: WO 2008/009739

(56) References cited:
- EP-A2- 0 144 644
- US-A- 3 708 306
- US-A- 5 384 136
- US-A1- 2004 241 257
- US-A1- 2006 159 724
- US-B1- 6 500 480
- ENZI G ET AL: "EFFECT OF A HYDROPHILIC MUCILAGE IN THE TREATMENT OF OBESE PATIENTS" PHARMATHERAPEUTICA, CLAYTON-WRAY PUBLICATIONS, LONDON, GB, vol. 2, no. 7, 1980, pages 421-428, XP009000477 ISSN: 0308-051X
- KENDALL, CYRIL WC: "The Health Benefits of Psyllium" CANADIAN JOURNAL OF DIETETIC PRACTICE AND RESEARCH, [Online] vol. 65, no. 3, 2004, XP002419116 Retrieved from the Internet: URL:http://www.kelloggnutrition.com/includ es/pdf/2004%20Fall%20DCIns_Psyllium.pdf> [retrieved on 2007-02-07]
- ANONYMOUS: "Monograph - Plantago ovata (Psyllium)" ALTERNATIVE MEDICINE REVIEW, [Online] vol. 7, no. 2, 2002, pages 155-159, XP002419117 Retrieved from the Internet: URL:http://www.thorne.com/media/psyllium_m onograph.pdf> [retrieved on 2007-02-07]

## Description

The pet-food industry produces two main categories of food: wet food and dry food.

Wet food usually has a moisture level of over 70% water while dry food rarely contains more than 12%. Wet food is distributed in the form of cans whereas dry food is in the form of granules, biscuits and, most commonly, extrudated nuggets.

Wet food primarily consists of meat, abattoir by-products and/or fish. Dry foods consist of cereals, various by-products of the food industry (flour, starch, sugar and oil producing industries), dried meat or fish meal, minerals (calcium carbonate, calcium phosphates, mineral trace elements), vitamins and flavourings.

This is why wet food has been traditionally regarded as more appetizing than dry food.

Nonetheless, the pet-food industry has succeeded in overcoming this disadvantage by making dry food more and more palatable through the choice of ingredients used, the technology for manufacturing nuggets and, most importantly, the use of new flavourings which dogs like and which stimulate their appetites.

The unexpected consequence of this success is that 20 to 25% of dogs are obese in the industrialized countries. Overeating gives dogs excess metabolisable energy which accumulates mainly in the form of fat.

Obesity leads to many pathological disorders in these animals, the most common of which are arthritic disease, diabetes and cardiovascular disease.

Many methods have been put forward to reduce overeating in dogs, for example:
- Rationing the amount of food: the daily amount of food given to the animal is reduced drastically but hunger makes them nervous and can lead to unpleasant and possibly even aggressive behaviour;
- formulation of low-calorie products, especially by reducing the amount of fat. However, a severe cut in fat quantities can cause pathological skin or cardiovascular disorders as a result of a deficiency in essential fatty acid, especially the omega-3 fatty acids;
- formulation of low-calorie products with a higher protein content in order to increase the protein/calorie ratio. However, this type of formula has little effect on the spontaneous consumption of food by dogs and obliges the owner to apply strict rationing which is often difficult to impose and has the same disadvantages as the quantity rationing approach described above.

The problems caused by these current methods of controlling the energy intake of dogs are aggravated when animals are bred in a confined environment, such as a town apartment.

Moreover, it is known that the sensation of hunger in dogs gives rise to complex physiological mechanisms brining into play many physico-chemical and intermediate chemico-biological receptors:
- oro-pharyngeal, gustative and olfactory receptors;
- physical receptors of the stomach and intestine;
- chemosensitive receptors of the intestine;
- porto-hepatic chemoreceptors sensitive to glycaemia or pyruvate concentration in the portal vein;
- gastro-intestinal hormones including cholecystokinine;
- pancreas hormones, glucagon and insulin.

US 2004/0241257 describes an animal food supplement for the treatment of colic and other gastrointestinal problems containing up to 95 weight % psyllium material.

EP 0144644A describes food products for use in the control of constipation and/or as a dietary aid in weight loss and maintenance, containing at least 10 to 80 weight % psyllium.

US 6,500,480 describes a dry ready-to-eat cereal product, containing more than 20 weight % psyllium.

US 5,384,136 relates to a dough product to lower serum cholesterol levels and increase dietary fibre containing about 1.0 to about 5.0 grams per ounce of psyllium.

Enzi et al, "Effect of a hydrophilic mucilage in the treatment of obese patients", Pharmatherapeutica, Clayton-Wray Publications, London, GB, vol 2, no. 7, 1980, pages 421-428, describes a crossover study in 22 obese patients to evaluate the effect of a hydrophilic mucilage associated with an 800 calorie hypoglucidic diet, as compared to diet alone, on body weight and on plasma lipid levels.

US 2006/159724 describes a low-glycemic nutritional supplement to be incorporated into the diet of an overweight or obese patient comprising low glycemic index ingredients including carbohydrate source, a source of protein and a source of fat and further comprising a source of green tea extract, a source of 5-hydroxy-tryptophan (5-HTP) and a source of chromium.

Butterwick and Hawthorne, American Society for Nutritional Sciences, 1998, 27715-27755 relates to advances in dietary management of obesity in dogs and cats.

Butterwick and Markwell, American Journal of Veterinary Research, 1997, Volume 8, No. 3, pages 272-276 relates to the effect of amount and type of dietary fiber on food intake in energy restricted dogs.

The Applicant has unexpectedly discovered that psyllium can reduce the appetite of dogs and their spontaneous consumption of food.

This is why this invention relates the use of psyllium seeds or parts of seeds to be incorporated into dry food for dogs in order to reduce the appetite of dogs, at a concentration of over 0.2%, preferably over 0.5% and below 4% by weight of said foodstuff.

Psyllium is a small seed produced by plants of the genus *Plantago*, principally *Plantago ovata* and *Plantago afra.* Psyllium seeds are very rich in soluble and insoluble fibres (65 % cellulose, hemicellulose and lignin, on the one hand, and 35% of gums, pectins and mucilage on the other). These fibres are found mainly in the tegument or seed « husk ».

The Applicant has found that the effect of psyllium on dogs' appetites can be obtained with the whole psyllium seed and more advantageously, with its tegument (husk). Seeds and teguments can therefore be combined in a mixture.

Experiments have shown that even relatively small amounts of psyllium seeds or parts of seeds are enough to produce the above-described effect.

Therefore the quantity by weight of psyllium or parts of psyllium seeds should be greater than 0.2% by weight of the food, preferably greater than 0.3% by weight and even more preferably greater than 0.5%.

Levels below 4% can be used within the scope of this invention.

It is also possible to use mixtures of psyllium seeds and teguments (husks).

This invention also relates to dry food for dogs aimed at reducing their appetites containing psyllium seeds or parts of seeds at a concentration greater than 0.2% by weight, preferably greater than 0.5% and below 4% by weight, these dry foods for dogs containing preferably at least one cereal, at least one animal or vegetable protein source and at least one animal or vegetable fat source.

Psyllium seeds or parts of seeds can be added to known dry food compositions for dogs. Given the relatively small quantity needed to produce a reduction in appetite, it is not necessary to significantly modify the compositions in question which can be obtained using any conventional process used in this industry.

Without knowing the exact mechanism of this effect, the Applicant has carried out the following non-exhaustive experiments in order to demonstrate the ability of psyllium to reduce spontaneous food consumption in dogs.

### EXPERIMENT 1

We formulated and manufactured by extrusion two nugget foods consisting of cereals, poultry meal, maize gluten, dried beetroot pulp, poultry fat, fish oil, calcium carbonate, flavouring for dogs and a « premix of vitamins and mineral trace elements ».

The two foods were formulated to contain:
- Moisture: maximum 9.5 %.
- Metabolisable energy: minimum 2 900 kcal/kg
- Proteins: minimum 30 %.
- Fats: minimum 7.5 %.
- Crude fibre: minimum 17.5 %
- Ashes: maximum 5.8 %
- Non-nitrogenated extract: q.s. [100 - (moisture+ protein + fats + crude fibre + ashes)] %

The first food formulated in this way was used as the control. The second food, designated as « Psyllium », contained 0.5% psyllium tegument (husk).

For the purposes of the experiment, we also tested a third commercial pet food, Hill's Canine r/d, currently used to control obesity in dogs.

These three foods were tested on three groups of 6 dogs, each group including 2 Shetland sheepdogs (average weight: 8.5 kg), 2 Breton Spaniels (average weight : 20 kg) and 2 Labradors (average weight: 43 kg).

Each animal was fed with its corresponding test food *ad libitum* every day for a limited period of 15 minutes. At the end of the meal, the amount of food consumed by each animal was carefully weighed. The experiment was repeated for 10 days.

Average food consumption over a 10-day period was as follows (in grams/dog/day):

| | |
|---|---|
| - Control: | 216.2 c |
| - Psyllium: | 172.3 a |
| - Hill's Canine r/d: | 201.7 b (a, b, c: significant difference at p < 0.05). |

The presence of psyllium therefore considerably reduced spontaneous consumption in dogs: by 20.3% with respect to the control and by 14.6% with respect to the reference commercial product.

In order to eliminate differences in consumption related to the large differences in dog size, the results were adjusted to the « metabolic weight » of each animal (in grams/kg weight ^{0.73}) :

| | |
|---|---|
| - Control: | 18.4 b |
| - Psyllium: | 15.2 a |
| - Hill's Canine r/d: | 21.0 c |

(a, b, c: significant difference at p < 0,05).

In terms of metabolic weight, spontaneous consumption in dogs was considerably reduced by psyllium: by 17.4% with respect to the control and by 27.6% with respect to the reference commercial product.

### EXPERIMENT 2

We formulated and manufactured by extrusion two nugget foods consisting of cereals, poultry meal, maize gluten, dried beetroot pulp, poultry fat, fish oil, soya oil, calcium carbonate, flavouring for dogs and a « premix of vitamins and mineral trace elements ».

The two foods were formulated to contain:
- Moisture: maximum 9.5 %.
- Metabolisable energy: minimum 3 450 kcal/kg
- Proteins: minimum 28 %.
- Fats: minimum 9 %.
- Crude fibre: minimum 7.9 %
- Ashes: maximum 4.7 %
- Non-nitrogenated extract: q.s. [100 - (moisture + protein + fats + crude fibre + ashes)] %

The first food formulated in this way was used as the control. The second food, designated as « Psyllium », contained 0.5% psyllium tegument (husk).

These two foods were tested on three groups of 6 dogs, each group including 2 Labradors (average weight: 33.5 kg), 2 German Shepherds (average weight : 29.5 kg) and 2 Boxers(average weight: 30.5 kg).

The two foods were distributed as follows: each animal was fed *ad libitum* for 15 minutes at 08, 09, 10 and 11 o'clock.

Average food consumption was as follows (in grams/kg weight^{0.73}) :

| | Control | Psyllium |
|---|---|---|
| 08 o'clock | 36.1 b | 31.1 a |
| 09 o'clock | 5.6 a | 3.5 a |
| 10 o'clock | 8.0 a | 7.2 a |
| 11 o'clock | 4.6 a | 5.1 a |
| In total | 54.3 b | 46.8 a |

(on the same a, b line: significant difference at p ≤ 0,05).

The reduction in food consumption in the presence of psyllium is significant at the first hour and at 3 hours. The presence of psyllium therefore greatly reduces spontaneous consumption by dogs, by 13.8% compared to the control.

### EXPERIMENT 3

We carried out the following experiment in order to establish whether these differences in consumption were due to the lack of palatability of psyllium:

105 dogs of various breeds and very different sizes were fed *ad libitum* simultaneously with two bowls, one containing « Psyllium » food and the other containing Hill's Canine r/d as used in Experiment 1. After 60 minutes, the bowls were removed and the food consumed weighed.

Average consumption expressed as a percentage of the total amount of the two foods consumed by each animal is as follows:

| | |
|---|---|
| - Psyllium: | 82 a |
| - Hill's Canine r/d: | 18 b |

(a, b: significant difference at p < 0,05).

This experiment shows that psyllium in no way diminishes appetite for the food containing it.

### EXPERIMENT 4

We carried out the following experiment in order to establish whether these differences in consumption were due to the lack of palatability of psyllium:

27 dogs of various breeds and very different sizes were fed *ad libitum* simultaneously with two bowls, one containing « Psyllium » food and the other containing the Control Food of Experiment 1. After 60 minutes, the bowls were removed and the food consumed weighed.

Average consumption expressed as a percentage of the total amount of the two foods consumed by each animal is as follows:

| | |
|---|---|
| - Psyllium: | 81 a |
| - Control: | 19 b |

(a, b: significant difference at p < 0,05).

This experiment shows that psyllium in no way diminishes appetite for the food containing it.

This set of four experiments shows that the presence of psyllium significantly reduces spontaneous consumption of food and that this reduction in consumption is not because psyllium unappetizing.

### EXPERIMENT 5

The three food types used in Experiment 1 were again given to the dogs used in the first experiment. However, the daily food ration was modified as follows: each animal was fed *ad libitum* for 15 minutes, at 8, 9, 10 and 11 o'clock in the morning.

Average consumption of each food was as follows (in grams/kilo weight^{0.73}):

| | Control | Psyllium | Hill's Canine r/d |
|---|---|---|---|
| 08 o'clock | 19.4 b | 14.1 a | 16.4 ab |
| 09 o'clock | 11.5 b | 5.3 a | 10.3 b |
| 10 o'clock | 9.2 b | 1.7 a | 7.0 b |
| 11 o'clock | 4.5 b | 0.6 a | 3.7 b |

(on the same a, b line: significant difference at p ≤ 0,05).

This effect of psyllium in decreasing food consumption lasts for at least 3 hours.

### EXPERIMENT 6

Experiment 5 was repeated but with food only given twice a day, at 8 o'clock in the morning and 3 o'clock in the afternoon.

Average consumption of each food was as follows (in grams/kilo weight^{0.73}) :

| | Control | Psyllium | Hill's Canine r/d |
|---|---|---|---|
| 08 o'clock | 21.8 b | 15.6 a | 19.8 b |
| 15 o'clock | 22.3 b | 16.0 a | 19.2 b |

(on the same a, b line: significant difference at p ≤ 0,05).

This effect of psyllium in decreasing food consumption lasts for at least 7 hours.

This set of experiments shows that the presence of psyllium in food significantly reduces spontaneous consumption by dogs and that this decrease is not due to the lack of palatability of psyllium.

## Claims

1. Use of psyllium seeds or parts of seeds incorporated into dry food for dogs, to reduce the appetite of dogs, at a concentration of over 0.2% and below 4% by weight of said foodstuff.

2. The use of psyllium according to claim 1, wherein the psyllium seeds or parts of seeds are at a concentration of over 0.5% by weight of the food.

3. The use according to claim 1 or claim 2, wherein psyllium is provided in the form of a mixture of seeds and teguments (husks).

4. The use according to anyone of claims 1 to 3, wherein the psyllium used is the tegument (psyllium husk) which is incorporated into the food at a concentration of over 0.3% by weight.

5. The use according to any one of claims 1 to 4, wherein the food contains at least one cereal, at least one animal or vegetable protein source and at least one animal or vegetable fat source.

6. A dry food for dogs to reduce the appetite of dogs which contains psyllium seeds or parts of seeds at a concentration of over 0.2% and below 4% by weight of said foodstuff:

7. The dry food for dogs according to claim 6, wherein the psyllium seeds or parts of seeds are at a concentration of over 0.5% by weight of the foodstuff.

8. The dry food for dogs according to claim 6 or claim 7, wherein psyllium is provided in the form of a mixture of seeds and teguments (husks).

9. The dry food for dogs according to any one of claims 6 to 8, wherein the psyllium used is the tegument (psyllium husk) which is incorporated into the food at a concentration of over 0.3% by weight.

10. The dry food for dogs according to any one of claims 6 to 9, wherein it contains at least one cereal, at least one animal or vegetable protein source and at least one animal or vegetable fat source.

## Patentansprüche

1. Verwendung von Psyllium-Samen oder -Samenteilen, die eingearbeitet sind in Trockenfutter für Hunde, um den Appetit von Hunden zu verringern, bei einer Konzentration von über 0,2 und unter 4 Gew.-% des Futtermittels.

2. Verwendung von Psyllium nach Anspruch 1, wobei die Psyllium-Samen oder -Samenteile bei einer Konzentration von über 0,5 Gew.-% des Futters vorliegen.

3. Verwendung nach Anspruch 1 oder Anspruch 2, wobei Psyllium in Form eines Gemisches aus Samen und Integumenten (Samenhülsen) bereitgestellt ist.

4. Verwendung nach einem der Ansprüche 1 bis 3, wobei das verwendete Psyllium das Integument (Psyllium-Samenhülse) ist, das in das Futter bei einer Konzentration von über 0,3 Gew.-% eingearbeitet ist.

5. Verwendung nach einem der Ansprüche 1 bis 4, wobei das Futter wenigstens eine Cerealie, wenigstens eine tierische oder pflanzliche Proteinquelle und wenigstens eine tierische oder pflanzliche Fettquelle enthält.

6. Trockenfutter für Hunde, um den Appetit von Hunden zu verringern, das Psyllium-Samen oder -Sammenteile bei einer Konzentration von über 0,2 und unter 4 Gew.-% des Futtermittels enthält.

7. Trockenfutter für Hunde nach Anspruch 6, wobei die Psyllium-Samen oder -Samenteile bei einer Konzentration von über 0,5 Gew.-% des Futtermittels vorliegen.

8. Trockenfutter für Hunde nach Anspruch 6 oder Anspruch 7, wobei Psyllium in der Form einer Mischung von Samen oder Integumenten (Samenhülsen) bereitgestellt ist.

9. Trockenfutter für Hunde nach einem der Ansprüche 6 bis 8, wobei das verwendete Psyllium das Integument (Psyllium-Samenhülse) ist, das in das Futter bei einer Konzentration von über 0,3 Gew.-% eingearbeitet ist.

10. Trockenfutter für Hunde nach einem der Ansprüche 6 bis 9, wobei es wenigstens eine Cerealie, wenigstens eine tierische oder pflanzliche Proteinquelle und wenigstens eine tierische oder pflanzliche Fettquelle enthält.

## Revendications

1. Utilisation de graines ou de parties de graines de psyllium incorporées dans un aliment sec pour chien en vue de diminuer l'appétit des chiens, à un taux supérieur à 0,2% et inférieur à 4 % en poids de l'aliment.

2. Utilisation selon la revendication 1, **caractérisée en ce que** les graines ou parties de graines de psyllium sont incorporées dans l'aliment à un taux supérieur à 0,5% en poids de l'aliment.

3. Utilisation selon l'une des revendications 1 et 2, **caractérisée en ce que** le psyllium est apporté par un mélange de graines et de téguments (coques).

4. Utilisation selon l'une des revendications 1 à 3, **caractérisée en ce que** la partie de psyllium utilisée est un tégument (coque de psyllium) et est incorporée dans l'aliment à un taux supérieur à 0,3% en poids.

5. Utilisation selon l'une quelconque des revendications 1 à 4, **caractérisé en ce qu'**il contient au moins une céréale, au moins une source de protéine animale ou végétale, au moins une source de graisse animale ou végétale.

6. Aliment sec pour chien en vue de diminuer l'appétit des chiens, lequel contient des graines ou parties de graines de psyllium, à un taux supérieur à 0,2% et inférieur à 4 % en poids de l'aliment.

7. Aliment sec pour chien selon la revendication 6, **caractérisé en ce qu'**il contient en poids plus de 0,5 % de graines ou parties de graines de psyllium.

8. Aliment sec pour chien selon la revendication 6 ou la revendications 7, dans lequel le psyllium est apporté par un mélange de graines et de téguments (coques)

9. Aliment sec pour chien selon l'une quelconque des revendications 6 à 8, dans lequel la partie de psyllium utilisée est un tégument (coque de psyllium) et est incorporée dans l'aliment à un taux supérieur à 0,3% en poids

10. Aliment sec pour chien selon l'une quelconque des revendications 6 à 9, selon lequel il contient au moins une céréale, au moins une source de protéine animale ou végétale, au moins une source de graisse animale ou végétale.
